# EUROPÄISCHE PATENTSCHRIFT

(11) **EP 0 434 010 B1**
(45) Veröffentlichungstag und Bekanntmachung des Hinweises auf die Patenterteilung: **23.03.1994**
(21) Anmeldenummer: 90124710.6
(22) Anmeldetag: 19.12.1990
(51) Int. Cl.: A61K 6/087, A61C 13/00, C09J 5/02, C09J 163/00

(54) **Haftmittlergemisch (Blend) für die zahnärtzliche Prothetik und dessen Verwendung als Dentalbindemittel**
Adhesion promoting composition (blend) for dental prosthetics and its use as dental adhesive
Composition (blend) à propriété adhésive pour prothèses dentaires et son utilisation comme adhésif dentaire

(30) Priorität: 22.12.1989 DD 336163
(43) Veröffentlichungstag der Anmeldung: 26.06.1991
(73) Patentinhaber: Vita Zahnfabrik H. Rauter GmbH & Co KG, D-79713 Bad Säckingen (DE)
(72) Erfinder: Müller, Wolf-Dieter, Dr., O-1185 Berlin (DE); Ludeck, Wolfgang, Dipl.-Ing., O-1136 Berlin (DE)
(74) Vertreter: Werner, Hans-Karsten, Dr.Dipl.-Chem.

(56) Entgegenhaltungen:
- EP-A- 0 077 096
- EP-A- 0 286 904
- DE-C- 3 617 127
- FR-A- 2 344 280
- US-A- 4 882 372

## Beschreibung

Die Erfindung betrifft einen Haftvermittler für die zahnärztliche Prothetik und dessen Verwendung als Dentalbindemittel, der speziell bei der Verblendung und Reparatur von Kronen, Brücken und Prothesen eingesetzt werden kann.

In der zahnärztlichen Prothetik werden für Verbundsysteme vorrangig Edelmetalle bzw. Nichtedelmetallegierungen eingesetzt, die mit geeigneten keramischen Substanzen bzw. insbesondere mit Kunststoffen verblendet werden. Sowohl bei Metall-Keramik als auch insbesondere bei Metall-Kunststoff-Restaurationen werden dabei zur Abdeckung der Farbe des Metalls und zur Erzielung der gewünschten Zahnfarbe der den Haftvermittler enthaltenden Grundierungsschicht Opakerfüllstoffe wie z.B. Titandioxid oder Zinkoxid sowie ggf. weitere Füllstoffe zugesetzt.

Im Falle von Metall-Kunststoff-Verbunden ist häufig die mit den verwendeten Haftvermittlern erzielbare Verbundfestigkeit zwischen beiden Werkstoffen ohnehin nicht befriedigend und wird bei zusätzlicher Verwendung von Opaker-Füllstoffen meistens soweit herabgesetzt, daß der Einbau von Retentionshilfen, z.B. in Form von Perlen, Schlaufen oder Netzen, erforderlich wird. Neben dem Aufwand sind der gestiegene Platzanspruch und auch die nicht gleichmäßige Wirksamkeit in der Randzone solcher Verbundsysteme als negativ zu betrachten.

Durch die Variation des Haftvermittlertyps und des Opaker- bzw. Füllstoffzusatzes wurde versucht, die genannten Nachteile zu eliminieren (DE-PS 30 01 616, DE-PS 21 62 608, DE-PS 33 32 179). Den Anforderungen an die Festigkeit und dauerhafte mediale Belastbarkeit der damit hergestellten Verbundsysteme konnte jedoch auch dadurch nicht ausreichend entsprochen werden.

Weiterhin hat sich gezeigt, daß Phosphon- oder Phosphorsäureesterverbindungen zwar häufig gute haftvermittelnde Eigenschaften besitzen, jedoch die Stabilität der damit hergestellten Verbundsysteme unter den medialen Bedingungen der Mundhöhle als nicht ausreichend anzusehen ist (DE-PS 28 18 068, DE-OS 27 11 234, DE-PS 27 39 282, US-PS 3 882 600).

Auch Haftvermittler auf der Basis von alpha-Cyanacrylaten haben sich als nicht ausreichend geeignet erwiesen. Weiterhin wurden zahlreiche Haftvermittler auf der Basis von speziellen polymerisierbaren Acrylsäureestern und/oder Methacrylsäureestern in Kombination mit ungesättigten OH- und/oder COOH-haltigen Monomeren für den Einsatz als Dentalbindemittel vorgeschlagen (DE-PS 25 37 528, DE-PS 33 32 179, JP-OS 14 318/1967, JP-OS 29 195/1970, DE-PS 36 17 127). Dabei müssen z.T. aufwendige Monomersynthesen durchgeführt und ein erhöhter Applikationsaufwand beim Einsatz dieser Systeme als Dentalbindemittel, insbesondere durch die erforderlichen, speziellen Polymerisationstechniken, erbracht werden. Über die damit erzielbaren Verbundfestigkeiten und die sonstigen Eigenschaften des damit hergestellten Prothetikmaterials werden keine Angaben gemacht. Es liegt jedoch die Vermutung nahe, daß die Haftvermittlerwirkung, insbesondere bei längerer medialer Belastung des genannten Prothetikmaterials in der Mundhöhle, nicht zufriedenstellend ist. Ein weiterer wesentlicher Nachteil dieser Variante besteht in der Neigung solcher polymerisierbarer Systeme zur Schrumpfung und damit zu starker Randspaltbildung bei dem damit hergestellten Prothetikmaterial. Es wurde auch schon ein Haftvermittler beschrieben, der viele der vorher genannten Mängel beseitigt, aber den Nachteil hat, daß trotz hoher Verbundfestigkeit, auch nach medialer Beansprchung, der Bruch nach der Belastung immer in der Grenzfläche zwischen dem opaken Haftvermittler und der Legierungsoberfläche erfolgt.

Aufgabe der Erfindung ist es, einen Haftvermittler für die zahnärztliche Prothetik zu entwickeln, der als Dentalbindemittel eingesetzt werden kann und dabei zu einer hohen Festigkeit im Verbund Edelmetall bzw. Edelmetallegierung bzw. Nichtedelmetall bzw. Nichtedelmetallegierung und Deckkunststoff (auf Polymethylmethacrylatbasis) führt, die auch nach längerer Beanspruchung unter medialer Belastung in der Mundhöhle erhalten bleibt.

Erfindungsgemäß wird die Aufgabe dadurch gelöst, daß der Haftvermittlergrundstoff aus einem Gemisch von einem mittelmolekularen Dianepoxidharz (Epoxidäquivalent >210, vorzugsweise 350) und einem Methyl/Ethylacrylat-Copolymeren (Molmasse Mn=5x10⁴ bis 1,5x10⁵; Säurezahl 23-100 mg KOH/g) besteht. Der Molmasseanteil des Epoxidharzes beträgt 50-90 Masse-%, der des Copolymeren 10-50 Masse-%. Für die Mischung mit Copolymeren der Säurezahl 23 beträgt das bevorzugte Masseverhältnis 70/30, bei Verwendung des Copolymeren mit der Säurezahl 62 beträgt das Masseverhältnis 60/40. Die Herstellung des Gemisches erfolgt durch Vermahlen in einer Kugelmühle, bevorzugt in einer Achatmühle.

Der erfindungsgemäße Haftvermittler kann technisch einfach und ökonomisch vorteilhaft als Dentalbindemittel in der zahnärztlichen Prothetik und entsprechend der dort hierzu üblicherweise angewendeten Praxis eingesetzt werden. Hierzu wird die Oberfläche des Metalls oder einer Legierung in einer für dentale Zwecke üblichen Zusammensetzung beruhenden Prothetikgrundgerüstes zunächst in an sich bekannter Weise, vorzugsweise durch Strahlen mit silanisiertem Quarz der Korngröße 160 bis 250 µm, vorbereitet.

Auf das so vorbehandelte Prothetikgrundgerüst wird das Dentalbindemittel (der erfindungsgemäß zusammengesetzte Haftvermittler) durch Streuen oder Sprühen aufgetragen.

Durch anschließendes Aufschmelzen bei 90 - 120°C, vorzugsweise 90°C, wird eine ca. 50 - 100 µm dicke Haftvermittlerschicht gebildet. Zur Erzielung einer optimalen Eigenfestigkeit der Haftvermittlerschicht ist eine Temperung bei 140 - 180°C, vorzugsweise bei 160°C, über 15 bis 90 Minuten, vorzugsweise 60 Minuten, erforderlich. Anschließend wird der endgültige Verbund mit dem jeweiligen Deckkunststoff durch Beschichten mit einer hierzu üblichen Mischung, z.B. bestehend aus PMMA, MMA und einem Initiatorsystem, und deren anschließende Polymerisation, z.B. mittels einer hydropneumatischen Heißpolymerisation, hergestellt. Der erfindungsgemäße Haftvermittler bewirkte bei der Verwendung als Dentalbindemittel überraschenderweise zum einen hervorragende Verbundfestigkeiten in dem damit hergestellten zahnärztlichen Prothetikmaterial, die auch bei medialer Beanspruchung unter den Bedingungen in der Mundhöhle lange erhalten blieben, und wies zum anderen trotz des Gehaltes an Epoxidverbindungen keinerlei physiologisch bedenkliche Nebenwirkungen auf.

Die Erfindung soll nachstehend an drei Ausführungsbeispielen näher erläutert werden.

### Ausführungsbeispiele

### Beispiel 1: Haftvermittlerzusammensetzung

60 Masseanteile in % Dianepoxidharz (Epoxidäquivalten 350), 40 Masseanteile in % carboxylgruppenmodifiziertes PMMA/EA-Copolymer (Säurezahl 62,0 mg KOH/g), 15 Minuten in einer Achatmühle vermahlen.

### Beispiel 2: Haftvermittlerzusammensetzung

70 Masseanteile in % Dianepoxidharz (Epoxidäquivalent 350), 30 Masseanteile in % carboxylgruppenmodifiziertes PMMA/EA-Copolymer (Säurezahl 23,0 mg KOH/g), 15 Minuten in einer Achatmühle vermahlen.

### Beispiel 3:

Ein zahnärztliches Prothetikgrundgerüst aus einer Ni-Cr-Mo-Al-Legierung der Zusammensetzung 69,6/16,7/5,6/4,2 Masseanteile in % wurde zunächst durch Sandstrahlen mit silaniertem, amorphem Quarz der Korngröße 160 bis 200 µm vorbehandelt. Auf das so vorbereitete Material wurde der Haftvermittler, das entsprechend der Zusammensetzung nach Beispiel 1 oder Beispiel 2 erhaltene Pulvergemisch, aufgestreut, bei 90°C aufgeschmolzen, so daß eine gleichmäßige Haftvermittlerschicht auf der Legierungsoberfläche gebildet und 60 Minuten bei 160°C vorgehärtet wird. Anschließend wurde der endgültige Verbund des so vorbereiteten Grundgerüstes in an sich bekannter Weise durch Beschichten mit einem Deckkunststoff einer hierzu üblichen Mischung, bestehend aus PMMA, MMA und einem Initiatorsystem, und deren hydropneumatischer Heißpolymerisation hergestellt. Es wurden Druck-Scher-Festigkeiten von 16,0 MPa nach der Herstellung sowie 14,5 bzw. 17,0 MPa in Simultationstests für die mediale Belastbarkeit unter Bedingungen der Mundhöhle nach 60 bzw. 10.000 Thermalzyklen zwischen 288 und 338 K im Wasserbad erhalten. Das sich nach der Druck-Scher-Belastung aufzeigende Bruchbild weist auf einen sehr festen Verbund zwischen Haftvermittler und Legierungsoberfläche hin, da die Bruchfläche im Bereich zwischen dem Haftvermittler und dem Deckkunststoff liegt. Somit wird die Legierungsoberfläche durch den Haftvermittler auch vor Angriffen durch korrosive Medien geschützt.

## Patentansprüche

1. Haftvermittler für die zanhärztliche Prothetik, dadurch gekennzeichnet, daß der Haftvermittler aus 50-90 Masseanteile in % eines Dianepoxidharzes (mit einem Epoxidäquivalent >210) und 10 - 50 Masseanteile in % eines auf Methyl-/Ethylacrylat basierenden carboxylgruppenhaltigen Copolymers (Molmasse Mn=5x10⁴ bis 1,5x10⁵ und einer Säurezahl von 7,0-100 mgKOH/g) besteht.

2. Haftvermittler nach Anspruch 1, dadurch gekennzeichnet, daß ein Dianepoxidharz mit einem Epoxidäquivalent von 350 und 60-70 Masseanteil in % eingesetzt wird.

3. Haftvermittler nach Anspruch 1, dadurch gekennzeichnet, daß das Copolymer bei einer Säurezahl von 62 mit 40 Masseanteilen in % und bei einer Säurezahl von 23 mit 30 Masseanteilen in % eingesetzt wird.

4. Verfahren zur Herstellung eines Haftvermittlers nach Ansprüche 1 bis 3, dadurch gekennzeichnet, daß die Bestandteile in einer Kugelmühle 15 Minuten gemischt werden.

5. Verfahren zur Anwendung eines Haftvermittlers nach Ansprüche 1 bis 4, dadurch gekennzeichnet, daß der Haftvermittler auf die Oberfläche aufgesprücht oder aufgespritzt wird.

6. Verfahren zur Anwendung eines Haftvermittlers nach Ansprüchen 1 bis 4, dadurch gekennzeichnet, daß der Haftvermittler bei 90 bis 120°C aufgeschmolzen und damit die Haftvermittlerschicht auf der Oberfläche gebildet wird.

7. Verfahren zur Anwendung eines Haftvermittlers nach Ansprüchen 1 bis 6, dadurch gekennzeichnet, daß der Haftvermittler anschließend bei 140 bis 180°C, vorzugsweise bei 160°C, 15 bis 90 Minuten, vorzugsweise 60 Minuten, vorgehärtet wird.

8. Verwendung des Haftvermittlers für die zahnärztliche Prothetik als Dentalbindemittel, dadurch gekennzeichnet, daß ein gemäß Ansprüchen 1 bis 7 zusammengesetzter Haftvermittler auf eine an sich bekannte Weise durch Sandstrahlen mit amorphem Quarz der Konrgröße 50,90 bis 160, 160 bis 200, 250, 300 bis 400 und 400 bis 500 µm vorbehandelte Oberfläche eines zahnärztlichen Prothetikgerüsts aus Edelmetall oder Nichtedelmetall, bzw. entsprechenden Legierungen, aufgebracht und mit heißhärtendem Deckkunststoff beschichtet wird.

## Claims

1. An adhesion promoter for dental prosthetics, characterized in that the adhesion promoter consists of an amount of from 50 to 90% by mass of a bisphenol A epoxide resin (having an epoxide equivalent of >210) and an amount of from 50 to 10% by mass of a copolymer based on methyl/ethlyl acrylate and containing carboxylic groups (molar mass Mₙ = 5 x 10⁴ to 1.5 x 10⁵ and an acid value of from 7.0 to 100 mg KOH/g).

2. The adhesion promoter according to claim 1, characterized in that a bisphenol A epoxide resin having an epoxide equivalent of 350 is used in an amount of from 60 to 70% by mass.

3. The adhesion promoter according to claim 1, characterized in that the copolymer is used in the case of an acid value of 62 in an amount of 40% by mass and in the case of an acid value of 23 in an amount of 30% by mass.

4. A process for producing an adhesion promoter according to claims 1 through 3, characterized in that the components are mixed in a ball mill for 15 minutes.

5. A process for the application of an adhesion promoter according to claims 1 through 4, characterized in that the adhesion promoter is atomized or sprayed onto the surface.

6. A process for the application of an adhesion promoter according to claims 1 through 4, characterized in that the adhesion promoter is melted at from 90 °C to 120 °C on the surface, thereby to form the adhesion-promoting layer.

7. A process for the application of an adhesion promoter according to claims 1 through 6, characterized in that the adhesion promoter is subsequently pre-cured at from 140 °C to 180 °C, and preferably at 160 °C, for 15 to 90 minutes, and preferably for 60 minutes.

8. Use of the adhesion promoter for dental prosthetics as a dental bonding agent, characterized in that an adhesion composed according to claims 1 through 7 is applied onto a surface of a dental prosthetics skeleton made of noble metal or non-noble metal or appropriate alloys, which surface has been pre-treated in a *per* *se* known manner by jet-blasting with amorphous quartz having particles sizes of 50, 90 to 160, 160 to 200, 250, 300 to 400 and 400 to 500 µm, and is then coated with a thermosetting synthetic topcoat material.

## Revendications

1. Agent adhésif pour prothèses dentaires, caractérisé en ce que l'agent adhésif est composé de 50 à 90 % en poids d'une résine de dianépoxy (équivalence en époxy >210) et de 10 à 50% en poids d'un copolymère contenant des groupes carboxyles, à base de méthyle/éthyle-acrylate (de masse moléculaire Mn = 5 x 10⁴ à 1,5 x 10⁵ et d'un indice d'acidité de 7,0 à 100 mg KOH/g).

2. Agent adhésif selon la revendication 1, caractérisé en ce qu'on utilise une résine de dianépoxy, ayant une équivalence en époxy de 350, et en proportion de 60 à 70% en poids.

3. Agent adhésif selon la revendication 1, caractérisé en ce qu'on utilise le copolymère ayant un indice d'acidité de 62 et en proportion de 40% en poids et ayant un indice d'acidité de 23 et 30% en poids.

4. Procédé de fabrication d'un agent adhésif selon les revendications 1 à 3, caractérisé en ce que les composants sont mélangés pendant 15 minutes dans un broyeur à billes.

5. Procédé d'application d'un agent adhésif selon les revendications 1 à 4 , caractérisé en ce que l'agent adhésif est épandu ou projeté sur la surface.

6. Procédé d'application d'un agent adhésif selon les revendications 1 à 4, caractérisé en ce que l'agent adhésif est fondu sur la surface à une température de 90 à 120°C, formant ainsi une couche adhésive sur la surface.

7. Procédé d'application d'un agent adhésif selon les revendications 1 à 6, caractérisé en ce que l'agent adhésif est durci au préalable, juste avant application, à une température de 140 à 190°C - de préférence à 160°C - pendant 15 à 90 minutes - de préférence pendant 60 minutes.

8. Application de l'agent adhésif à des prothèses dentaires comme liant dentaire, caractérisé en ce qu'un agent adhésif constitué selon les revendications 1 à 7, est appliqué sur la surface d'une prothèse dentaire en métal précieux, métal non-précieux ou en alliage de ces métaux - traitée au préalable selon une méthode connue par sablage avec du quartz amorphe à granulation de 50, 90 à 160, de 160 à 200, 250, de 300 à 400 et de 400 à 500 µm - et recouverte d'une couche de matière synthétique durcie à chaud.
